# DEMANDE DE BREVET EUROPEEN

(11) **EP 3 266 324 A1**
(43) Date de publication de la demande: **10.01.2018**
(21) Numéro de dépôt: 17180170.7
(22) Date de dépôt: 07.07.2017
(51) Int. Cl.: A41C 1/10

(54) **CEINTURE POUR FEMME ENCEINTE**

(30) Priorité: 08.07.2016 FR 1656589
(71) Demandeur: BABYMOOV GROUP, 63100 Clermont-Ferrand (FR)
(72) Inventeur: WINDENBERGER, Laurent, 63000 CLERMONT-FERRAND (FR)
(74) Mandataire: Lavoix

(57) **Abrégé**

Cette ceinture (1) comporte, d'une part, une partie ventrale (10), qui inclut une portion centrale (10C) et deux portions latérales (10G, 10D) disposées de part et d'autre de la portion centrale, de sorte que, lorsque la ceinture est portée par une femme enceinte (F), la portion centrale recouvre la région médiane du ventre de la femme tandis que les portions latérales recouvrent les flancs respectivement gauche et droit du ventre de la femme, et, d'autre part, une partie dorsale (20) qui est adaptée pour s'attacher dans le dos de la femme. Pour que la ceinture permette à la femme de pouvoir dormir confortablement et sereinement, chaque portion latérale de la partie ventrale intègre un coussin de calage au niveau duquel cette portion latérale est plus épaisse que la portion centrale de manière que, lorsque la femme est couchée sur son côté, gauche ou droit, correspondant au flanc de son ventre recouvert par cette portion latérale, le coussin de calage supporte le ventre de la femme en comblant l'espace entre le flanc correspondant du ventre et la surface (S) sur laquelle la femme est couchée, chaque portion latérale de la partie ventrale comprenant au moins un panneau textile (16), auquel est rapporté le coussin de calage correspondant (14) et auquel sont solidarisées à demeure la portion centrale et la partie dorsale. Afin que la ceinture soit pratique et simple à utiliser, l'invention prévoit que le ou les panneaux textiles (16) de chaque portion latérale (10G, 10D) de la partie ventrale (10) forment une poche (15) dans laquelle le coussin de calage correspondant (14) est reçu de manière amovible.

## Description

La présente invention concerne une ceinture pour femme enceinte.

Pour les femmes enceintes, on connait des ceintures qui se présentent sous la forme d'une large bande textile élastique, que la femme porte tout autour d'elle, en recouvrant son ventre, ce dernier étant de plus en plus arrondi au cours de la grossesse, en particulier dans la seconde moitié de celle-ci. Ces bandes élastiques sont soit réalisées d'une seule pièce fermée sur elle-même, qui est souvent appelée « bandeau de grossesse » et qui est disponible en plusieurs tailles pour correspondre aux diverses morphologies des femmes et à la grosseur de leur ventre, soit réalisées sous forme de bandes dites de maintien, conçues pour, de manière réversible, se fermer et s'ajuster autour de la femme, en s'attachant notamment dans le dos de la femme. Dans tous les cas, ces ceintures de grossesse permettent, de par leur élasticité adaptée, de maintenir et soutenir le ventre de la femme, afin de soulager le dos de celle-ci lors des activités de la femme.

Plus généralement, les vêtements de soutien de maternité sont conçus pour soulager la douleur du dos au niveau de la région lombaire et/ou de la région pelvienne. Ces vêtements peuvent être classés en quatre catégories principales : 1-ceintures lombaires, 2-caleçons, 3-berceaux et 4-supports de torse. La ceinture de grossesse se décline selon plusieurs modèles. Il existe tout d'abord les ceintures que l'on porte sous le ventre, au niveau du bassin, et qui aident au maintien du ventre. Il existe aussi la ceinture de grossesse qui part du bassin et couvre tout ou partie du ventre. Néanmoins, toutes ces ceintures se portent le jour, en particulier lorsque la femme est débout, puisqu'elles sont conçues pour reporter le poids du ventre de la femme, en répartissant ce poids tout autour de la femme, de manière à corriger ainsi la posture générale du corps de la femme afin d'éviter des douleurs associées.

Même si les ceintures de grossesse connues apportent un réel soulagement postural pour la femme enceinte en position débout, elles sont totalement inefficaces pour agir sur le ventre de la femme enceinte lorsque celle-ci est en position couchée pour dormir. En effet, en fin de grossesse, plus généralement lors de la seconde moitié de la grossesse, la taille du ventre de la femme impose à celle-ci de dormir sur le côté, étant remarqué que la position allongée sur le dos est inconfortable pour la femme et le foetus. Lorsque la femme enceinte est couchée sur le côté, son ventre tend, par l'effet de son poids, à déséquilibrer le corps de la femme vers l'avant, ce qui conduit à comprimer le ventre et donc à gêner la femme et le foetus. Le sommeil de la femme enceinte est donc souvent perturbé. Pour contourner cette difficulté et améliorer quelque peu la qualité du sommeil, il est connu de chercher à placer, entre le flanc du ventre de la femme couchée sur le côté et la surface sur laquelle elle est couchée, un coussin. Cependant, cette solution basée sur un tel coussin d'appoint s'avère limitée, même dans le cas où la forme de ce coussin d'appoint a été prévue dans l'optique de soulager le ventre des femmes enceintes en position couchée sur le côté. En effet, la mise en place de ce coussin d'appoint est souvent difficile car il s'agit de le placer au plus près du flanc du ventre tout en tenant compte de la souplesse inhérente de la surface, de type matelas, sur laquelle la femme est couchée. En particulier, si le coussin d'appoint est trop appuyé contre le flanc du ventre, il est source d'inconfort. De plus, dès que la femme bouge dans son sommeil, ne serait-ce que de quelques centimètres, le coussin d'appoint n'est plus positionné de manière adéquate, ce qui le rend inutile et conduit rapidement à ce que la femme enceinte se réveille pour réajuster la position du coussin.

US 5 492 496, qui peut être considéré comme l'état de la technique le plus proche de l'invention, divulgue une ceinture pour femme enceinte, répondant à la problématique expliquée jusqu'ici. Cette ceinture comporte, à la fois, une partie dorsale permettant d'attacher la ceinture dans le dos de la femme enceinte, et une partie ventrale incluant, de part et d'autre d'une portion centrale, des portions latérales, respectivement gauche et droite. Chacune de ces portions latérales comprend un panneau textile extérieur et un panneau textile intérieur, entre lesquels est enfermé à demeure un empilement de couches de rembourrage, chacun de ces deux empilements ayant un effet de calage pour le flanc, gauche ou droit, du ventre de la femme enceinte. Même si cette ceinture permet à la femme enceinte de supporter son ventre lorsqu'elle est couchée sur le côté, cette ceinture est peu pratique à utiliser du fait de la structure à la fois empilée et non accessible des couches de remplissage précitées. En particulier, l'effet de calage latéral procuré par cette ceinture n'est pas réglable et la ceinture n'est pas lavable.

De son côté, US 5 953 742 divulgue un dispositif de support abdominal qui comporte une bande élastique, un support gauche et un support droit. Chacun des supports comporte un bloc, en forme de coin et constitué de mousse polyuréthane. Chaque bloc est reçu dans un étui textile qui n'est pas intégré solidairement à la bande élastique, mais qui est prévu pour être accroché de manière détachable à cette bande élastique par un système de type VELCRO (marque déposée). Le but de la présente invention est de proposer une ceinture pour femme enceinte qui soit plus simple et plus pratique.

A cet effet, l'invention a pour objet une ceinture pour femme enceinte, telle que définie à la revendication 1.

Ainsi, l'invention s'apparente à une ceinture de sommeil pour femme enceinte, dans le sens où la ceinture conforme à l'invention intègre deux coussins de calage du ventre de la femme enceinte, qui agissent sur celui-ci, en le supportant, lorsque la femme est couchée sur le côté, typiquement pour dormir. Au sein de la ceinture conforme à l'invention, ces deux coussins sont agencés de manière à couvrir exclusivement les flancs latéraux, respectivement gauche et droit, du ventre de la femme enceinte lorsque celle-ci porte la ceinture, de sorte que lorsque la femme enceinte s'allonge sur son côté gauche ou sur son côté droit, l'un ou l'autre des deux coussins se retrouve automatiquement interposé entre le flanc correspondant de son ventre et la surface, typiquement le matelas, sur laquelle la femme s'allonge. De plus, chaque coussin de calage est dimensionné, notamment en épaisseur, pour conférer à la portion de la ceinture, où le coussin est intégré, une épaisseur suffisamment importante pour combler l'espace entre le flanc correspondant du ventre et la surface sur laquelle la femme est couchée sur le côté. Il en résulte que chaque coussin supporte latéralement le ventre de la femme et stabilise ainsi le corps de la femme en évitant que le flanc correspondant du ventre soit en porte à faux par rapport à la surface sur laquelle la femme est couchée sur le côté. Comme la ceinture est attachée autour du corps de la femme et qu'elle suit les mouvements du corps de la femme, cette dernière peut bouger dans son sommeil, voire se retourner et changer de côté sur lequel elle est allongée, tout en conservant le bénéfice de l'effet de support et de stabilisation procuré par les coussins de calage de la ceinture conforme à l'invention. Selon l'invention, les coussins de calage sont intégrés de façon amovible au reste de la ceinture, en étant respectivement reçus dans des poches textiles formés directement par les portions latérales de la partie ventrale de la ceinture. Le ou les panneaux textiles qui forment chacune de ces poches sont solidarisés à demeure, par exemple par des coutures, à la portion centrale de la partie ventrale et à la partie dorsale, tout en donnant accès, par une ouverture refermable, à l'intérieur de la poche pour atteindre le coussin de calage associé afin de pouvoir le retirer en totalité de la ceinture, en particulier à des fins de nettoyage, et afin de pouvoir le remettre en place avant réutilisation de la ceinture. En pratique, la forme de réalisation des coussins de calage, en particulier leur matière et leur profil, n'est pas limitative de l'invention du moment qu'ils soient amovibles et qu'ils assurent l'effet de support des flancs du ventre. La ceinture conforme à l'invention permet ainsi à la femme enceinte de dormir sereinement, et ce de manière particulièrement confortable et efficace, grâce au fait que la ceinture s'ajuste, notamment par sa partie dorsale et par, avantageusement, la portion centrale de sa partie ventrale, au corps de la femme enceinte, et grâce au fait que les coussins de calage de la ceinture sont maintenus en place dans les poches, par le reste de la ceinture, contre les flancs latéraux du ventre. La ceinture conforme à l'invention est également très pratique, en étant notamment facile à nettoyer après retrait des coussins, et en offrant la possibilité d'interchanger les coussins entre différentes tailles et/ou différentes caractéristiques, telles que la fermeté ou autre, par exemple liées à la matière des coussins.

Des caractéristiques additionnelles avantageuses de la ceinture conforme à l'invention sont spécifiées aux revendications dépendantes.

L'invention sera mieux comprise à la lecture de la description qui va suivre, donnée uniquement à titre d'exemple et faite en se référant aux dessins sur lesquels :
- les figures 1 et 2 sont des vues schématiques en perspective, sous des angles d'observation respectifs différents, d'une femme enceinte portant une ceinture conforme à l'invention ;
- la figure 3 est une vue schématique en élévation de la ceinture des figures 1 et 2, montrée déployée à plat ;
- la figure 4 est une section schématique sur la ligne IV-IV à la figure 3 ;
- la figure 5 est une vue similaire aux figures 1 et 2, montrant l'utilisation de la ceinture par la femme enceinte couchée sur son côté droit ; et
- la figure 6 est une section schématique dans le plan VI de la figure 5.

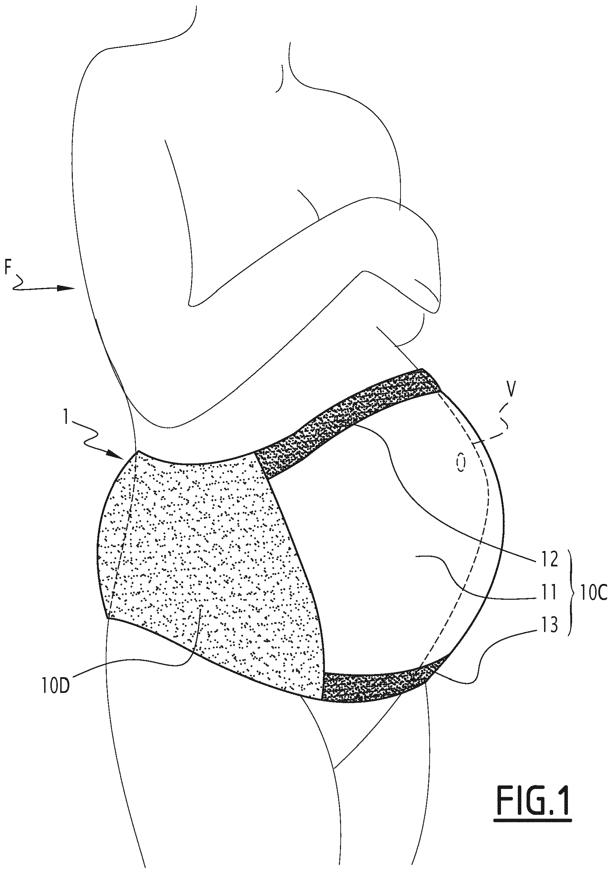

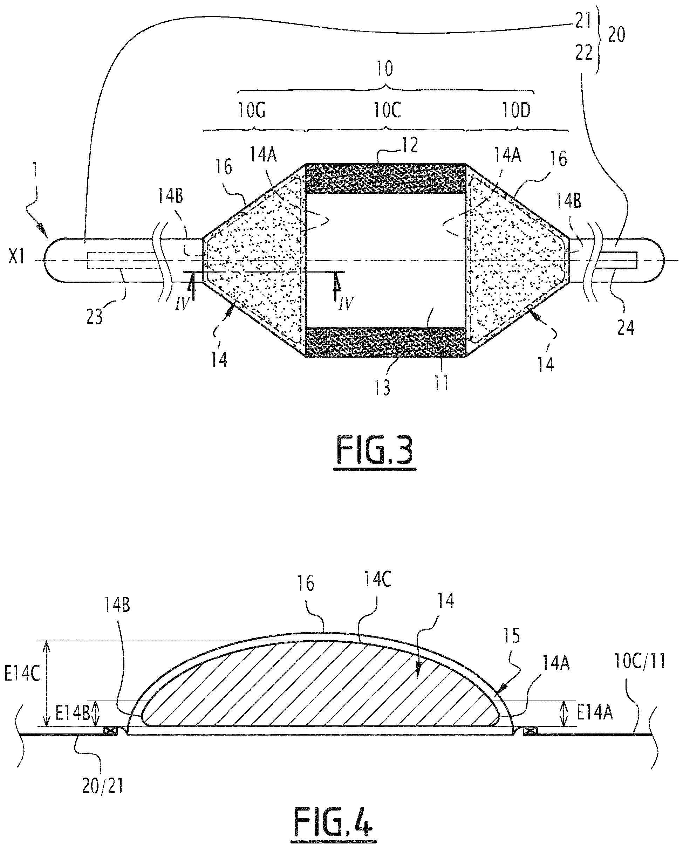

Sur les figures 1 à 6 est représentée une ceinture pour femme enceinte 1. Sur les figures 1, 2, 5 et 6, la ceinture 1 est portée par une femme enceinte F dont la grossesse est dans la seconde moitié, voire le troisième trimestre. Sur les figures 3 et 4, la ceinture 1 est représentée non portée, en étant considérée étalée à plat.

Comme bien visible sur les figures 3 et 4, la ceinture 1 se présente globalement sous la forme d'une bande allongée dont la direction longitudinale est référencée X1. Lorsque la ceinture 1 n'est pas portée et qu'elle est déployée à plat comme sur les figures 3 et 4, la direction longitudinale X1 est sensiblement rectiligne, tandis que lorsque la ceinture 1 est portée par la femme F comme sur les figures 1, 2, 5 et 6, la ceinture 1 entoure tout le corps de la femme F, à hauteur de son ventre V, la direction longitudinale X1 courant tout autour du corps de la femme F en suivant le profil de son ventre V et de son dos D.

Eu égard à la forme générale de bande de la ceinture 1, cette dernière présente une face intérieure et une face extérieure : la face intérieure, qui est dirigée vers le lecteur sur la figure 3 et qui est dirigée vers le bas sur la figure 4, est tournée vers le corps de la femme F lorsque la ceinture 1 est portée, tandis que la face extérieure, qui est dirigée vers le haut sur la figure 4, est tournée à l'opposé du corps de la femme F lorsque la ceinture est portée. Par la suite, on définit l'épaisseur comme étant la dimension de la ceinture entre ses faces intérieure et extérieure, la direction associée à cette épaisseur étant naturellement perpendiculaire à la direction longitudinale X1. Quant à la troisième dimension de la ceinture 1, c'est-à-dire sa dimension autre que son épaisseur et sa longueur (sa dimension selon la direction longitudinale X1), cette troisième dimension constitue la largeur de la ceinture 1.

Comme bien visible sur les figures 1 et 3, la ceinture 1 se répartit en une partie ventrale 10, qui lorsque la ceinture 1 est portée par la femme F, recouvre son ventre V, et en une partie dorsale 20 qui, lorsque la ceinture est portée, recouvre le dos D de la femme F. Ces parties ventrale 10 et dorsale 20 de la ceinture 1 vont être progressivement décrites plus en détail.

Comme bien visible sur la figure 3, la partie ventrale 10 inclut une portion centrale 10C et deux portions latérales qui sont disposées de part et d'autre, dans la direction longitudinale X1, de la portion centrale 10C, à savoir une portion latérale gauche 10G et une portion latérale droite 10D. Lorsque la ceinture 1 est portée, la portion centrale 10C recouvre la région médiane VM du ventre V de la femme F, c'est-à-dire la région de son ventre où sont son nombril et les zones entourant le nombril tant latéralement que verticalement, comme montré sur les figures 1, 5 et 6, tandis que, d'une part, la portion latérale gauche 10G recouvre le flanc gauche VG du ventre V et, d'autre part, la portion latérale droite 10D recouvre le flanc droit VD du ventre V, comme montré sur les figures 1, 2, 5 et 6. Autrement dit, lorsque la ceinture 1 est portée par la femme F, son ventre V est recouvert à l'avant par la portion centrale 10C et latéralement par les portions 10G et 10D.

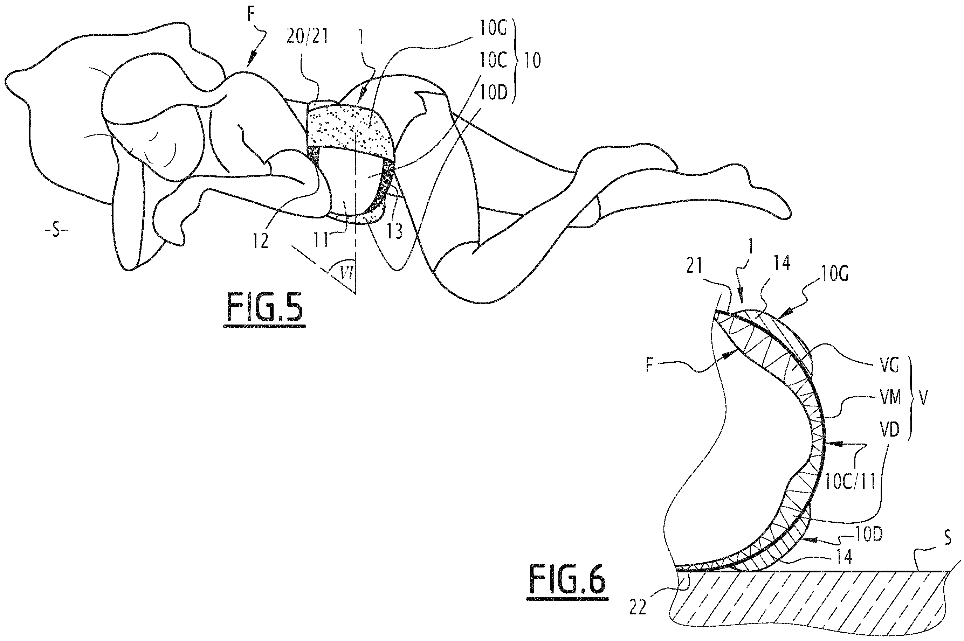

Dans la forme de réalisation considérée sur les figures, la portion centrale 10C de la partie ventrale 10 de la ceinture 1 comprend un panneau principal 11 qui est bordé, au-dessus et au-dessous de lui, par des bandeaux respectivement supérieur 12 et inférieur 13. Lorsque la ceinture 1 est portée par la femme F, le panneau principal 11 recouvre la zone ombilicale de la région médiane VM de son ventre V, tandis que les bandeaux supérieur 12 et inférieur 13 sont situés sensiblement à la hauteur de, respectivement, la zone épigastrique et la zone hypogastrique de la région médiane VM du ventre V, comme bien visible sur les figures 1 et 5. Le panneau principal 11 est prévu extensible, en étant par exemple et de manière non limitative réalisé en jersey, de manière à s'adapter, par extension souple, à l'évolution du ventre V de la femme F au cours de la grossesse. Chacun des bandeaux 12 et 13 relie l'une à l'autre les portions latérales 10G et 10D de manière élastique, en étant par exemple et de manière non limitative réalisés au moins partiellement en une matière élastomérique. Avantageusement, le module d'élasticité des bandeaux 12 et 13 est plus grand que celui du panneau principal 11 de manière que, lorsque la ceinture 1 est portée, la portion centrale 10C est maintenue en place sur le ventre V de la femme F, tant vers la gauche et la droite que vers le haut et le bas, davantage par les bandeaux 12 et 13 que par le panneau principal 11. En d'autres termes, tant en sollicitation latérale que verticale, les bandeaux 12 et 13 contribuent, de manière prépondérante au sein de la portion centrale 10C, au maintien en place de cette portion centrale 10C et, par-là, de la ceinture 1 vis-à-vis du ventre V de la femme F, tandis que le panneau principal 11 épouse de manière confortable et légère la région médiane VM du ventre V de la femme F.

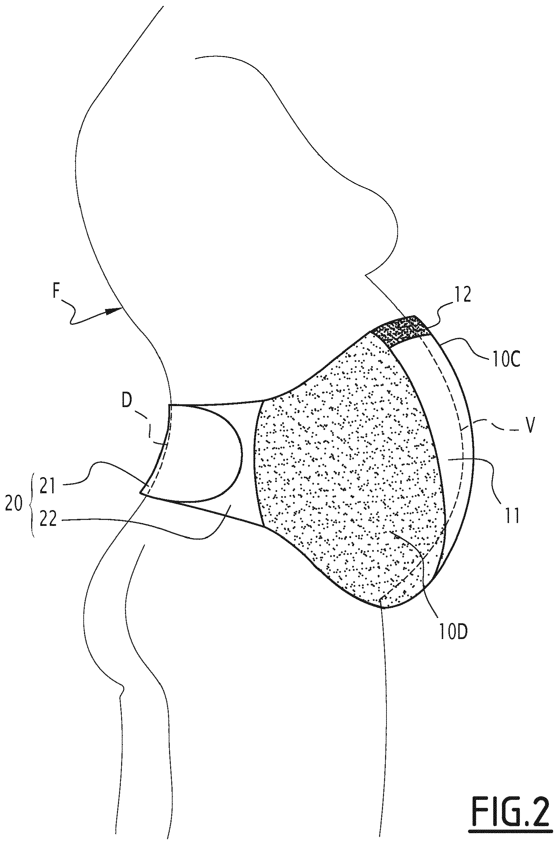

Egalement dans l'exemple de réalisation considéré ici, la partie dorsale 20 est répartie de part et d'autre, dans la direction longitudinale X1, de la partie ventrale 10. Ainsi, comme bien visible sur la figure 3, la partie dorsale 20 comprend une sangle gauche 21, qui s'étend depuis la portion latérale gauche 10G de la partie ventrale 10, et une sangle droite 22 qui s'étend depuis la portion latérale droite 10D de la partie ventrale 10. Les sangles 21 et 22 sont pourvues respectivement de parties complémentaires d'un système d'attache, connu en soi, permettant d'attacher les sangles 21 et 22 l'une à l'autre dans le dos D de la femme F lorsque la ceinture 1 est portée, comme montré sur la figure 2. A titre d'exemple non limitatif, la sangle 21 est pourvue, sur sa face extérieure, d'un ruban mâle 23, muni de crochets textiles, tandis que la sangle 22 est pourvue, sur sa face intérieure, d'un ruban femelle 24, muni de bouclettes textiles auxquelles les crochets précités peuvent s'auto-agripper lorsque la face extérieure de la sangle 21 est mise en recouvrement par la face intérieure de la sangle 22, dans le dos D de la femme F. Autrement dit, dans cet exemple, le système d'attache est de type Velcro (marque déposée). Bien entendu, d'autres formes de réalisation sont envisageables pour le système d'attache porté par les sangles 21 et 22. Plus généralement, on comprend que la partie dorsale 20 de la ceinture 1 présente des aménagements permettant d'attacher cette partie dorsale 20 dans le dos D de la femme F, en fermant la ceinture 1 sur elle-même autour du corps de la femme F et en ajustant cette ceinture dans sa direction longitudinale X1 pour la serrer autour du corps de la femme F.

Comme bien visible sur les figures 3 et 4, chacune des portions latérales 10G et 10D est pourvue d'un coussin 14 qui, par définition, est souple et forme une surépaisseur pour la portion latérale 10G, 10D comparativement au reste de la ceinture 1, en particulier comparativement à la portion centrale 10C. Ainsi, au niveau de chaque coussin 14, la portion latérale correspondante 10G, 10D présente une épaisseur supérieure à celle de la portion centrale 10C, valant au moins deux fois, voire trois, voire quatre fois, voire cinq fois, voire dix fois l'épaisseur de la portion centrale 10C. En pratique, le coussin 14 est dimensionné, notamment en épaisseur, de sorte que l'épaisseur de la portion latérale correspondante 10G, 10D soit suffisante pour combler l'espace entre le flanc correspondant VG, VD du ventre V de la femme F portant la ceinture 1 et la surface S sur laquelle la femme F est couchée sur le côté, comme montré sur les figures 5 et 6. Ainsi, lorsque la ceinture 1 est portée par la femme F alors que celle-ci est couchée sur le côté sur la surface S, le coussin 14 est interposé, par son épaisseur, entre cette surface S et le flanc correspondant VG, VD du ventre V et supporte donc latéralement le ventre V en le stabilisant vis-à-vis de la surface S. Ainsi, les coussins 14 peuvent être qualifiés de coussins de calage.

Suivant un dimensionnement préférentiel, qui garantit un bon effet de support et de stabilisation du ventre V, chaque coussin 14 est dimensionné de façon que la partie latérale correspondante 10G, 10D présente, au niveau de son coussin 14, une épaisseur valant, au moins localement, plus de trois centimètres.

Chaque coussin 14 est intégré à la portion latérale correspondante 10G et 10D de la partie ventrale 10 de la ceinture 1, dans le sens où les deux coussins 14 sont des parties intégrantes de la ceinture 1 en restant, en service, solidaires du reste de cette ceinture. En d'autres termes, lorsque la ceinture 1 est portée, les coussins 14 sont solidaires de la ceinture 1 au niveau, respectivement, de la portion latérale gauche 10G et de la portion latérale droite 10D. Ceci étant, cette intégration des coussins 14 aux portions latérales 10G et 10D de la partie ventrale 10 est prévue amovible, par exemple à des fins de nettoyage, étant entendu que le dégagement des coussins 14 vis-à-vis des portions latérales 10G et 10D n'est possible que lorsque la ceinture 1 n'est pas fonctionnelle. A cet effet, chaque coussin 14 est reçu, de manière amovible, dans une poche 15 délimitée par la portion latérale correspondante 10G, 10D, cette poche étant normalement fermée lorsque la ceinture 1 est fonctionnelle, en particulier lorsqu'elle est portée, tandis que cette poche peut être ouverte, par tout système approprié, pour accéder au coussin 14 et ainsi le dégager puis le remettre en place lorsque la ceinture 1 n'est pas fonctionnelle. En pratique, cette poche 15 est formée par un panneau textile de la portion latérale correspondante 10G, 10D ou par un ensemble de panneaux textiles de cette portion, assemblés les uns aux autres, ce ou ces panneaux textiles portant la référence 16 sur les figures 3 et 4.

Un des intérêts de ce ou ces panneaux textiles 16 est qu'il(s) est/sont facilement solidarisé(s) à demeure à la portion centrale 10C de la partie ventrale 10 et à la partie dorsale 20, en étant par exemple, mais de manière non limitative, couturé(s) au panneau principal 11, aux bandeaux 12 et 13 et aux sangles 21 et 22.

En pratique, le ou les matériaux constitutifs des coussins de calage 14 ne sont pas limitatifs de l'invention. Suivant une forme de réalisation préférentielle, chaque coussin 14 est constitué d'un corps qui est amovible d'un seul tenant par rapport au reste de la ceinture et qui est complémentaire de la poche 15 correspondante afin d'y être reçu de manière ajustée. Chaque coussin peut ainsi être retiré dans son ensemble en un seul geste, tout en pouvant ensuite être aussi facilement remis en place dans la poche en y étant positionné de manière précise et calée.

Selon une forme de réalisation avantageuse, le corps constituant chaque coussin 14 est réalisé sous forme d'un bloc en mousse à mémoire de forme, cette mousse étant connue en tant que telle et étant un composé chimique à base de polyuréthane, mélangée à d'autres composés qui en augmentent la densité et la viscosité pour conférer à la mousse un comportement viscoélastique. Ceci étant, d'autres matériaux et/ou d'autres structures peuvent être envisagés pour le corps constituant chaque coussin 14. Une première possibilité est que, plutôt que d'être en mousse à mémoire de forme, un bloc formant le corps de chaque coussin 14 peut être en mousse conventionnelle ou en tissu tridimensionnel. Une deuxième possibilité est que le corps de chaque coussin 14 comporte une housse, notamment textile, remplie d'un rembourrage tel que de la ouate, du coton, des plumes, du papier par exemple sous forme de confetti, des billes, des microbilles, des fibres végétales, des fibres synthétiques, des poils d'animaux, du sable, des noyaux, en particulier des noyaux de cerise, et des graines, en particulier des graines de colza, de lavande et/ou de lin. Une troisième possibilité est que le corps de chaque coussin 14 comporte un sac étanche rempli d'air et/ou de liquide, tel que de l'eau, et/ou de gel.

Suivant une disposition optionnelle, qui est mise en oeuvre dans l'exemple de réalisation considéré sur les figures et qui est particulièrement visible sur la figure 4, chaque coussin 14 n'a pas une épaisseur constante, mais une épaisseur qui varie dans la direction longitudinale X1. Plus précisément, comme illustré par la figure 4, lorsqu'on parcourt chaque coussin 14 dans la direction longitudinale X1, depuis son bord 14A tourné vers la portion centrale 10C jusqu'à son bord opposé 14B, l'épaisseur du coussin 14 passe d'une valeur minimale E14A au niveau du bord 14A, à, progressivement, une valeur maximale E14C au niveau d'une région centrale 14C du coussin 14, puis à, progressivement, une valeur moindre E14B au niveau du bord 14B, correspondant sensiblement à la valeur minimale précitée E14A. De cette façon, la surépaisseur, induite par chaque coussin 14, s'établit de manière progressive au niveau des portions latérales 10G et 10D, vis-à-vis de la portion centrale 10C et de la partie dorsale 20 lorsqu'on parcourt la ceinture 1 dans sa direction longitudinale X1. Toute sensation de butée ou de « marche », à la jonction entre les portions latérales 10G et 10D et le reste de la ceinture 1, est ainsi évitée ou, toute le moins, atténuée. Avantageusement, pour le confort de la femme F, cette variation d'épaisseur de chaque coussin 14 induit une conformation plus bombée pour la face extérieure du coussin 14 que pour sa face intérieure, cette dernière pouvant même, le cas échéant, être prévue sensiblement plane lorsque le coussin est au repos, comme envisagé sur la figure 4.

Suivant une autre disposition optionnelle, qui est également mise en oeuvre dans l'exemple de réalisation considéré sur les figures, la partie dorsale 20, en particulier ses sangles 21 et 22, est prévue moins large que la portion centrale 10C de la partie ventrale 10. De cette façon, l'étendue verticale de la zone du dos D recouverte par la partie dorsale 20 lorsque la ceinture 1 est portée, est minimisée, c'est-à-dire limitée comparativement à l'étendue de la zone du ventre V recouverte par la portion centrale 10C de la partie ventrale 10. On cherche ainsi à augmenter le confort de la partie dorsale 20, notamment en évitant que cette partie dorsale 20 ne donne chaud ou limite la mobilité de la femme F dans son sommeil. La différence de largeur entre la partie dorsale 20 et la portion centrale 10C de la partie ventrale 10 est accommodée par les portions latérales 10G et 10D de la partie ventrale 10, ces portions latérales présentant chacune globalement la forme d'un trapèze, comme bien visible sur la figure 3. Chaque coussin 14 est alors prévu lui aussi avec un profil en trapèze ou, plus généralement, prévu de manière que son bord 14A soit plus large que son bord 14B, sans dégrader l'effet de support et de stabilisation conféré par les coussins 14 vis-à-vis des flancs VG et VD du ventre V de la femme F.

## Revendications

1. Ceinture pour femme enceinte, comportant :
- une partie ventrale (10) qui inclut une portion centrale (10C), ainsi que deux portions latérales (10G, 10D) disposées de part et d'autre de la portion centrale, de sorte que, lorsque la ceinture (1) est portée par une femme enceinte (F), la portion centrale recouvre la région médiane (VM) du ventre (V) de la femme tandis que les portions latérales recouvrent les flancs respectivement gauche (VG) et droit (VD) du ventre de la femme, et
- une partie dorsale (20) qui est adaptée pour s'attacher dans le dos de la femme,
dans laquelle chaque portion latérale (10G, 10D) de la partie ventrale (10) intègre un coussin de calage (14) au niveau duquel cette portion latérale est plus épaisse que la portion centrale (10C) de manière que, lorsque la femme (F) est couchée sur son côté, gauche ou droit, correspondant au flanc (VG, VD) de son ventre (V) recouvert par cette portion latérale, le coussin de calage supporte le ventre de la femme en comblant l'espace entre le flanc correspondant du ventre et la surface (S) sur laquelle la femme est couchée, et
dans laquelle chaque portion latérale (10G, 10D) de la partie ventrale (10) comprend au moins un panneau textile (16), auquel est rapporté le coussin de calage correspondant (14) et auquel sont solidarisées à demeure la portion centrale (10C) et la partie dorsale (20),
**caractérisée en ce que** le ou les panneaux textiles (16) de chaque portion latérale (10G, 10D) de la partie ventrale (10) forment une poche (15) dans laquelle le coussin de calage correspondant (14) est reçu de manière amovible.

2. Ceinture suivant la revendication 1, **caractérisée en ce que** chaque portion latérale (10G, 10D) de la partie ventrale (10) présente, au niveau de son coussin de calage (14), une épaisseur valant, au moins localement, plus de trois centimètres.

3. Ceinture suivant l'une des revendications 1 ou 2, **caractérisée en ce que** chaque coussin de calage (14) est constitué d'un corps qui est amovible d'un seul tenant par rapport au reste de la ceinture et qui est complémentaire de la poche correspondante (15) pour y être reçu de manière ajustée.

4. Ceinture suivant la revendication 3, **caractérisée en ce que** chaque coussin de calage (14) est réalisé sous forme d'un bloc en mousse à mémoire de forme.

5. Ceinture suivant la revendication 3, **caractérisée en ce que** chaque coussin de calage (14) comprend une housse remplie d'un rembourrage.

6. Ceinture suivant la revendication 3, **caractérisée en ce que** chaque coussin de calage (14) comprend un sac étanche rempli d'air, de liquide et/ou de gel.

7. Ceinture suivant l'une quelconque des revendications précédentes, **caractérisée en ce que** chaque coussin de calage (14) présente une épaisseur (E14A, E14B, E14C) qui augmente progressivement puis diminue progressivement lorsqu'on parcourt ce coussin de calage depuis son bord (14A), tourné vers la portion centrale (10C), jusqu'à son bord (14B) tourné à l'opposé de la portion centrale.

8. Ceinture suivant la revendication 7, **caractérisée en ce que** chaque coussin de calage (14) présente une face extérieure qui est conformée de manière plus bombée qu'une face intérieure du coussin de calage.

9. Ceinture suivant l'une quelconque des revendications précédentes, **caractérisée en ce que** la partie dorsale (20) est moins large que la portion centrale (10C) de la partie ventrale (10), et **en ce que** chaque portion latérale (10G, 10D) de la partie ventrale (10) accommode la différence de largeur entre la portion centrale et la partie dorsale, chaque coussin de calage (14) ayant son bord (14A), tourné vers la portion central, qui est plus large que son bord (14B) tourné à l'opposé de la portion centrale.

10. Ceinture suivant l'une quelconque des revendications précédentes, **caractérisée en ce que** la portion centrale (10C) de la partie ventrale (10) comprend :
- un panneau principal (11), qui est extensible de manière à s'adapter à l'évolution du ventre (V) de la femme (F) au cours de la grossesse, et
- deux bandeaux (12, 13), qui bordent le panneau principal, en étant situés respectivement au-dessus et au-dessous de ce panneau principal, et qui relient chacun les portions latérales (10G, 10D) l'une à l'autre de manière élastique,
et **en ce que** le module d'élasticité des bandeaux est plus grand que celui du panneau principal de manière que la portion centrale soit maintenue en place sur le ventre de la femme davantage par les bandeaux que par le panneau principal.

11. Ceinture suivant l'une quelconque des revendications précédentes, **caractérisée en ce que** la partie dorsale (20) comprend deux sangles (21, 22), qui s'étendent respectivement depuis l'une et depuis l'autre des portions latérales (10G, 10D) de la partie ventrale (10) et qui sont pourvues de parties respectives (23, 24) d'un système d'attache.

12. Ceinture suivant l'une quelconque des revendications précédentes, **caractérisée en ce que** la portion centrale (10C) de la partie ventrale (10) et la partie dorsale (20) sont couturées au ou aux panneaux textiles de chaque portion latérale (10G, 10D).

13. Ceinture suivant les revendications 10, 11 et 12 prises ensemble, **caractérisée en ce que** le panneau principal (11) et les bandeaux (12, 13) de la portion centrale (10C) de la partie ventrale (10), ainsi que les deux sangles (21, 22) de la partie dorsale (20) sont couturés au ou aux panneaux textiles (16) respectifs des portions latérales (10G, 10D).
